# EUROPÄISCHE PATENTSCHRIFT

(11) **EP 1 319 377 B1**
(45) Veröffentlichungstag und Bekanntmachung des Hinweises auf die Patenterteilung: **18.04.2007**
(21) Anmeldenummer: 02025454.6
(22) Anmeldetag: 15.11.2002
(51) Int. Cl.: A61F 5/058

(54) **Vorrichtung zur Stützung und Stabilisierung eines Verletzten**
Device for supporting and stabilizing an injured person
Dispositif servant à supporter et à stabiliser un blessé

(30) Priorität: 12.12.2001 AT 19412001
(43) Veröffentlichungstag der Anmeldung: 18.06.2003
(73) Patentinhaber: Kohlbrat & Bunz Gesellschaft m.b.H, 5550 Radstadt (AT)
(72) Erfinder: Rugfelt, Hakan, 23941 Falsderbo (SE)
(74) Vertreter: Torggler, Paul Norbert

(56) Entgegenhaltungen:
- WO-A-01/30280
- CH-A- 661 204
- NL-A- 7 101 392
- US-B1- 6 251 065

## Beschreibung

Die Erfindung betrifft eine Vorrichtung zur Stützung und Stabilisierung eines Verletzten, mit einem flexiblen, am Verletzten festlegbaren Folienelement, das einen luftdichten, evakuierbaren Innenraum einschließt, in dem ein Einsatzkörper aus luftdurchlässigen, flexiblen Materialbahnen angeordnet ist, der lose Partikel enthaltende Kammern aufweist.

Eine derartige Vorrichtung ist beispielsweise der WO 01/30280 zu entnehmen. Diese zeigt eine hauptsächlich als Oberkörperstütz- und -stabilisierungsweste ausgebildete Vorrichtung, in deren Einsatzkörper mit Partikel gefüllte Kammern in Umfangsrichtung, bei sitzender Position also horizontal um den Oberkörper verlaufen. Die Kammern sind durch Querstege so geteilt, daß sie einander in evakuiertem Zustand teilweise überlappen. Durch die Unterteilung wird verhindert, daß bei der Handhabung der Vorrichtung die bei Nichtverwendung unter Normaldruck beweglichen Partikel senkrecht zu den Querstegen zusammenrutschen, sodaß kein Teilbereich ohne Partikel ist. In Längsrichtung der Querstege ist die Beweglichkeit jedoch vorhanden und wird nur durch die geringe Kammernbreite verringert. Dennoch ist es meist erforderlich, die Partikel vor dem Anlegen an den Verletzten so gut es geht händisch zu verteilen, was Zeit kostet. Im evakuierten Zustand ist die Stabilität der Vorrichtung in Längsrichtung der Kammern gut, in Längsrichtung des Verletzten hingegen schlechter, da die Partikelpackung oftmals durch die Knickzonen bildenden Querstege unterbrochen ist.

Die CH 661 204 zeigt eine Stabilisierungsvorrichtung für den gesamten Körper, deren Kammern in Richtung der Wirbelsäule verlaufen. Die oben beschriebenen Nachteile treten daher jeweils um 90° versetzt auf. Die losen Partikel rutschen bei der Handhabung in den Kopf- oder Fußbereich, und die Querstabilität ist schlechter. Hiezu kommt noch ein weiterer, für den Verletzten nachteiliger Effekt, nämlich die beim Evakuieren durch die Verdichtung der Partikelpackung in Richtung der Wirbelsäule beträchtliche Längenschrumpfung im Zentimeterbereich, die gefährliche Kräfte auf die verletzte Wirbelsäule ausübt.

Die Erfindung hat es sich nun zur Aufgabe gestellt, eine Vorrichtung der eingangs genannten Art zu schaffen, die verringerte Längenschrumpfung aufweist. Weiters soll auch eine verbesserte Stabilität in Längs- und in Querrichtung gegeben sein. Erreicht wird dies dadurch, daß die Kammern in geschlossenen Taschen ausgebildet sind, die einander in Längsrichtung des Einsatzkörpers überlappen. Auf diese Weise werden durchgehende Längskammern vermieden, und die schuppen- bzw. dachziegelartige Überlappung der Taschen stellt die Stabilität der evakuierten Vorrichtung sicher.

Bevorzugt ist vorgesehen, daß die Taschen einander etwa über die Hälfte überlappen, wobei es günstig ist, wenn der Querrand jeder dritten Tasche den dazu parallelen Querrand jeder ersten Tasche überlagert. In dieser Ausführung liegen über die Länge der Vorrichtung gesehen, an jeder Stelle zwei Taschen übereinander, und in den Übergangsbereichen entlang der Querränder jeweils drei Taschen.

Eine weitere bevorzugte Ausführung sieht vor, daß die Taschen zwei zueinander parallele Ränder aufweisen, die mit den anderen Rändern nichtrechte Winkel einschließen. Die Winkel betragen insbesondere 90° ± 45°. Somit ist einerseits die Bewegung der Partikel sowohl in der Längsrichtung als auch in der Querrichtung mehrfach unterbrochen, und andererseits die Stabilität in diesen beiden Richtungen erhöht, da die Knickzonen schräg ausgerichtet sind. Das Zusammenrutschen der losen Partikel bei der Handhabung erfolgt im wesentlichen ebenfalls schräg, wobei aber die Taschen einen zur Rutschrichtung spitzwinkeligen Rand aufweisen, und die Füllung daher beim Zusammenrutschen aufgrund der konischen Begrenzung über eine größere Höhe erhalten bleibt. Ein füllungsloser Bereich ist dadurch kleiner.

Auch die Schrumpfung beim Evakuieren ist wesentlich verringert, da sowohl in Längsals auch in Querrichtung des Einsatzkörpers wirksame Unterbrechungen gegeben sind, die die Verdichtung der Partikelpackungen unterbrechen.

Der Einsatzkörper besteht vorzugsweise aus textilen Materialbahnen, beispielsweise Geweben, Vliesen od. dgl., die die entsprechende Luftdurchlässigkeit aufweisen, und wird befüllt und entlang seiner Umfangsränder oder mittels dort fixierter Befestigungslaschen zwischen zwei luftdichten Folien des mit einem Ventil versehenen Folienelementes fixiert. Die textilen Materialbahnen können entlang ihrer Ränder und entlang der Längs- und Querränder des Einsatzkörpers teilweise überlappend vernäht sein.

Die Kammern sind insbesondere durch mindestens eine Quernaht oder einen Quersteg gebildet, die bzw. der senkrecht zu den zueinander parallelen Rändern verläuft. Wenn schräg verlaufende Taschen durch eine Quernaht oder einen Quersteg unterteilt sind, ergeben sich weitere spitze Winkel, wodurch der beschriebene Effekt erhöht wird und füllungslose Bereiche weiter verkleinert werden. Der händische Ausgleich der Füllung vor dem Evakuieren wird dadurch auf ein Minimum verringert bzw. ist gegebenenfalls nicht notwendig, da schlechter gefüllte Taschenbereiche von besser gefüllten überdeckt werden.

Nachstehend wird nun die Erfindung an Hand der Figuren der beiliegenden Zeichnung näher beschrieben, ohne darauf beschränkt zu sein. Es zeigen
Fig. 1 eine schematische Schrägansicht einer ersten Ausführung eines Einsatzkörpers für eine Ganzkörper-Vakuummatratze,
Fig. 2 eine schematische Draufsicht auf eine zweite Ausführung,
Fig. 3 einen Längsschnitt nach der Linie III-III durch den Einsatzkörper und
Fig. 4 einen Querschnitt nach der Linie IV-IV der Fig. 2.

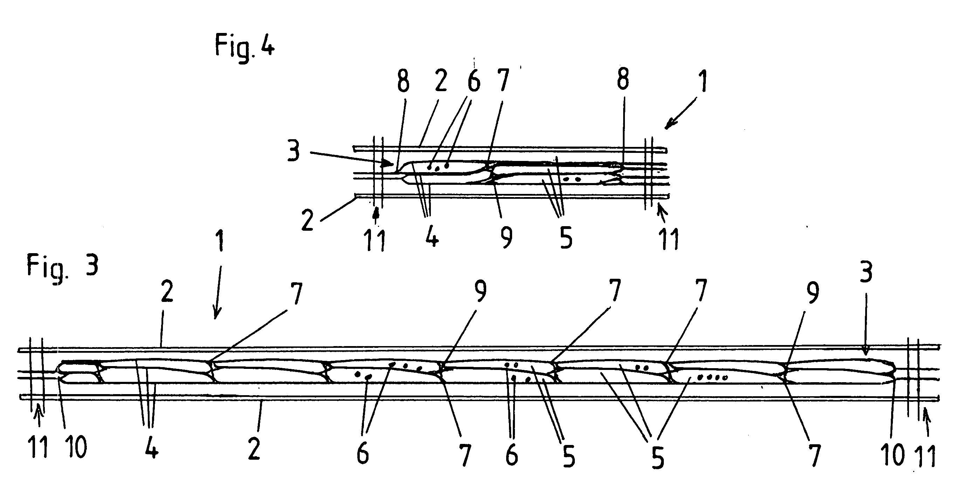

Ein Einsatzkörper 3 für eine Vorrichtung zur Stützung und Stabilisierung eines Verletzten umfaßt ein äußeres, flexibles Folienelement 1 aus luftdichten, entlang ihrer Ränder verschweißten Folien 2, insbesondere aus einem Polyvinylchlorid oder Polyurethan, die z.B. durch ein Polyestergewebe verstärkt sind, und die in den Fig. 3 und 4 angedeutet sind. Zwischen den beiden Folien 2 ist ein Einsatzkörper 3 angeordnet, dessen Randstreifen 11 direkt oder über geeignete Bänder 13 zwischen den Rändern der beiden Folien 2 fixiert sind. Eine der Folien 2 weist ein Ventil auf, über das der Innenraum des Folienelementes evakuiert werden kann. Der Einsatzkörper 3 besteht aus luftdurchlässigen, flexiblen Materialbahnen, insbesondere textilen Bahnen, aus denen Taschen 5 gebildet sind, die entlang der Ränder 7, 8 vernäht sind.

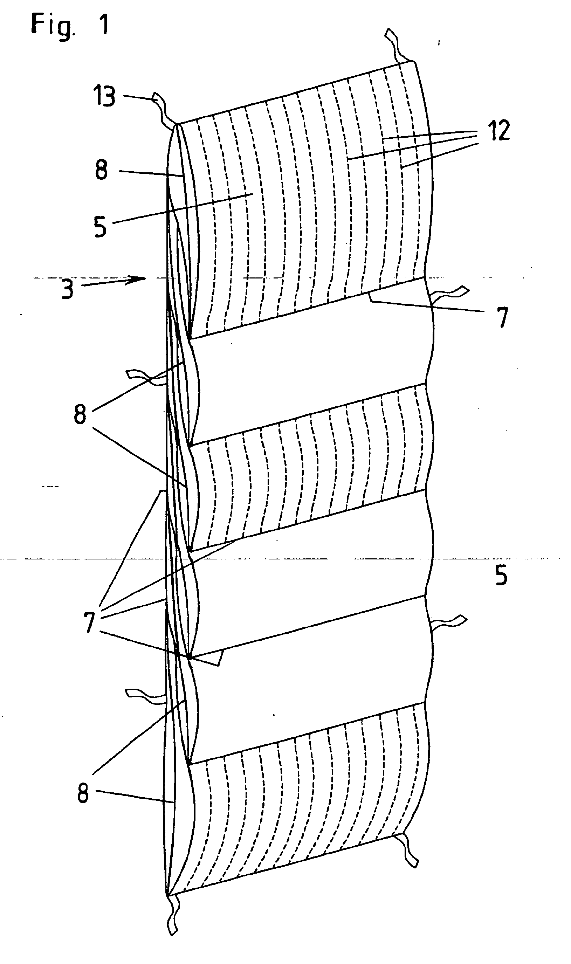

Gemäß Fig. 1 sind die Taschen 5 rechteckig und überlappen einander in der Weise, daß der in der Zeichnung untere Querrand 7 jeder Tasche 5 tiefer liegt als der obere Querrand 7 der übernächsten Tasche 5. Es wechseln daher beide Bereiche 16 mit zwei und schmale Bereiche 17 mit drei einander überlappenden Taschen 5 ab. An den Längsrändern 8 sind die einzelnen Taschen 5 miteinander verbunden, und mit den Bändern 13 zur Befestigung des Einsatzkörpers 3 zwischen den Folien 2 versehen. Die Taschen 5 sind durch mehrere in Längsrichtung verlaufende, sich senkrecht zu den Querrändern 7 erstreckende, strichliert gezeichnete Querstege 12 in Kammern unterteilt, in denen lose Partikel 6, beispielsweise Polystyrolkugeln enthalten sind.

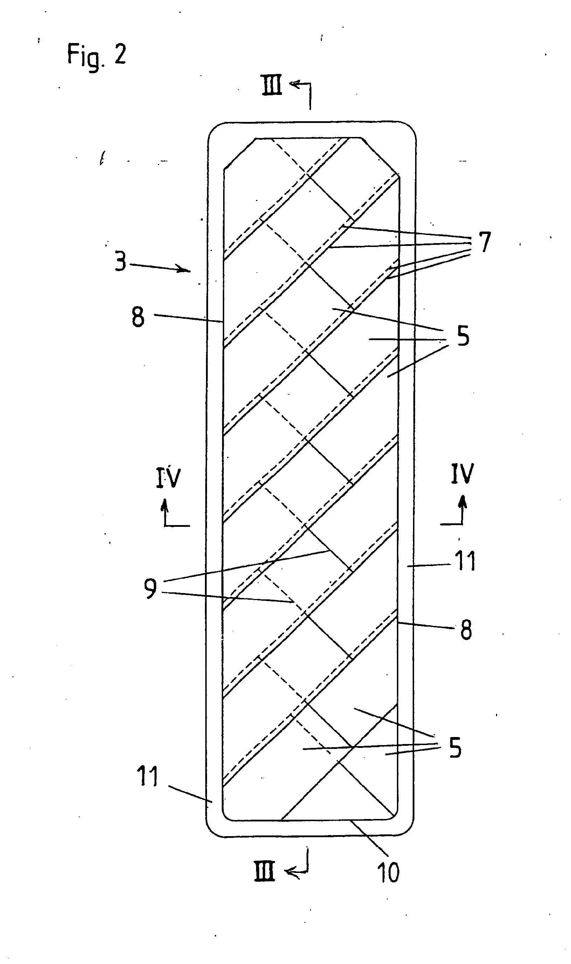

In der zweiten Ausführung nach den Fig. 2 bis 4 weisen die Taschen 5 zwei zueinander parallele Ränder 7 auf, die sich schräg, insbesondere unter 45°, zu den beiden anderen, ebenfalls zueinander parallelen Rändern 8 erstrecken. Die Ränder 7, 8 schließen daher jeweils Winkel von 45° und 135° ein. Die Taschen 5 überlappen einander um etwas mehr als die Hälfte, so daß auch hier grundsätzlich zwei, und an den Übergängen entlang der schrägen Ränder 7 jeweils drei Taschen 5 übereinander liegen, wie aus den Fig. 3 und 4 ersichtlich ist. Die Taschen 5 dieser Ausführung weisen mit der Ausnahme der obersten und untersten Tasche die Form eines Parallelogramms auf, wobei sie miteinander nur in den Randstreifen 11 außerhalb der in Längsrichtung verlaufenden Ränder 8 verbunden sind. Die beiden obersten Taschen und die unterste Tasche, die auch durch Quernähte 10 begrenzt werden, sind im wesentlichen dreieckig bzw. trapezförmig. Um das Volumen der Taschen 5 zu verringern, sind sie durch eine etwa mittig verlaufende Quernaht 9 geteilt. Die Quernaht 9 ist senkrecht zu den Rändern 7 gezeigt, kann aber auch senkrecht oder parallel zu den Außenrändern 8 verlaufen. Anstelle der Quernähte 9 könnten auch Querstege vorgesehen werden.

Die Ränder 7, 8 jeder Tasche 5 schließen mindestens einen Winkel ein, der zur Beibehaltung einer gleichmäßigen Partikelverteilung beiträgt. Wird das Folienelement 1 beim Transport aufrecht gehalten, so können die losen Partikel 6 zwar verrutschen, durch die spitzen Winkel ist aber das Aufnahmevolumen stark eingeschränkt, sodaß sich nur ein kleinerer Teil der Partikel 6 randnah anhäufen kann. Die Quernähte 9 und die Taschenüberlappung tragen ebenfalls dazu bei, die Gesamtverteilung der Partikel 6 innerhalb des Einsatzkörpers 3 zu begünstigen, sodaß im Anwendungsfall, in dem generell rasch behandelt werden muß, wesentlich weniger Zeit erforderlich ist, um eventuell zu dünn mit Partikeln bestückten Bereichen händisch einen Ausgleich zu erzielen, indem das Folienelement geschüttelt und plattgestrichen wird.

Die Schnitte der Figuren 3 und 4 zeigen weiters auch, daß durch die Schrägstellung der Taschen der Einsatzkörper 3 gleichzeitig in der Längs- und in der Querrichtung versteift wird, da Knickbereiche höchstens entlang der schrägen Ränder 7 entstehen können.

## Patentansprüche

1. Vorrichtung zur Stützung und Stabilisierung eines Verletzten, mit einem flexiblen, am Verletzten festlegbaren Folienelement (1), das einen luftdichten, evakuierbaren Innenraum einschließt, in dem ein Einsatzkörper (3) aus luftdurchlässigen, flexiblen Materialbahnen (4) angeordnet ist, der lose Partikel (6) enthaltende Kammern aufweist, **dadurch gekennzeichnet, daß** die Kammern in geschlossenen Taschen (5) ausgebildet sind, die einander in Längsrichtung des Einsatzkörpers überlappen.

2. Vorrichtung nach Anspruch 1, **dadurch gekennzeichnet, daß** die Taschen (5) einander etwa über die Hälfte überlappen.

3. Vorrichtung nach Anspruch 1, **dadurch gekennzeichnet, daß** der Querrand (7) jeder dritten Tasche (5) den dazu parallelen Querrand (7) jeder ersten Tasche (5) überlagert.

4. Vorrichtung nach einem der Ansprüche 1 bis 3, **dadurch gekennzeichnet, daß** die Taschen (5) zwei zueinander parallele Ränder (7) aufweisen, die mit den anderen Rändern (8) nichtrechte Winkel einschließen.

5. Vorrichtung nach Anspruch 4, **dadurch gekennzeichnet, daß** die zueinander parallelen Ränder (7) unter 45° zur Längs- und Querrichtung des Folienelementes (1) verlaufen.

6. Vorrichtung nach Anspruch 4 oder 5, **dadurch gekennzeichnet, daß** die Taschen (5) die Form eines Parallelogramms aufweisen.

7. Vorrichtung nach Anspruch 4 oder 5, **dadurch gekennzeichnet, daß** die Taschen (5) die Form eines Trapezes aufweisen.

8. Vorrichtung nach einem der Ansprüche 1 bis 7, **dadurch gekennzeichnet, daß** die Taschen (5) aus textilen Bahnen hergestellt und entlang ihrer Ränder (7, 8) vernäht sind.

9. Vorrichtung nach einem der Ansprüche 1 bis 8, **dadurch gekennzeichnet, daß** die Taschen (5) entlang ihrer sich in Längsrichtung des Einsatzkörpers (3) erstreckenden Ränder (8) miteinander verbunden sind.

10. Vorrichtung nach einem der Ansprüche 1 bis 9, **dadurch gekennzeichnet, daß** die Kammern durch zumindest eine die Taschen (5) unterteilende Quernaht (9) gebildet sind, die senkrecht zu den zueinander parallelen Rändern (7) verläuft.

11. Vorrichtung nach einem der Ansprüche 1 bis 9, **dadurch gekennzeichnet, daß** die Kammern durch zumindest einen die Taschen unterteilenden Quersteg gebildet sind, der senkrecht zu den zueinander parallelen Rändern (7) verläuft.

## Claims

1. An arrangement for supporting and stabilising an injured person comprising a flexible film element (1) which can be secured to the injured person and which encloses an air-tight evacuatable internal space in which is arranged an insert body (3) of air-permeable flexible material webs (4), which has chambers containing loose particles (6), **characterised in that** the chambers are provided in closed pockets (5) which overlap each other in the longitudinal direction of the insert body.

2. An arrangement according to claim 1 **characterised in that** the pockets (5) overlap each other approximately over half.

3. An arrangement according to claim 1 **characterised in that** the transverse edge (7) of each third pocket (5) overlies the transverse edge (7) parallel thereto of each first pocket (5).

4. An arrangement according to one of claims 1 to 3 **characterised in that** the pockets (5) have two mutually parallel edges (7) which include right angles to the other edges (8).

5. An arrangement according to claim 4 **characterised in that** the mutually parallel edges (7) extend at 45° relative to the longitudinal and transverse direction of the film element (1).

6. An arrangement according to claim 4 or claim 5 **characterised in that** the pockets (5) are in the form of a parallelogram.

7. An arrangement according to claim 4 or claim 5 **characterised in that** the pockets (5) are in the form of a trapezium.

8. An arrangement according to one of claims 1 to 7 **characterised in that** the pockets (5) are produced from textile webs and are sewn along their edges (7, 8).

9. An arrangement according to one of claims 1 to 8 **characterised in that** the pockets (5) are connected together along their edges (8) extending in the longitudinal direction of the insert body (3).

10. An arrangement according to one of claims 1 to 9 **characterised in that** the chambers are formed by at least one transverse seam (9) which subdivides the pockets (5) and which extends perpendicularly to the mutually parallel edges (7).

11. An arrangement according to one of claims 1 to 9 **characterised in that** the chambers are formed by at least one transverse limb which subdivides the pockets and which extends perpendicularly to the mutually parallel edges (7).

## Revendications

1. Dispositif pour le soutien et la stabilisation d'un blessé, avec un élément de feuille flexible (1) pouvant être fixé sur le blessé qui contient un espace intérieur étanche à l'air dans lequel le vide peut être fait, dans lequel est placé un corps d'insertion (3) constitué de bandes de matière flexibles (4) perméables à l'air qui comporte des compartiments contenant des particules en vrac (6), **caractérisé en ce que** les compartiments sont réalisés dans des poches fermées (5) qui se chevauchent dans la direction longitudinale du corps d'insertion.

2. Dispositif selon la revendication 1, **caractérisé en ce que** les poches (5) se chevauchent à peu près sur la moitié.

3. Dispositif selon la revendication 1, **caractérisé en ce que** le bord transversal (7) de chaque troisième poche (5) est superposé au bord transversal parallèle à ce dernier (7) de chaque première poche (5).

4. Dispositif selon l'une quelconque des revendications 1 à 3, **caractérisé en ce que** les poches (5) comportent deux bords transversaux (7) parallèles l'un à l'autre qui incluent des angles non droits avec les autres bords (8).

5. Dispositif selon la revendication 4, **caractérisé en ce que** les bords (7) parallèles l'un à l'autre s'étendent sous un angle de 45° par rapport à la direction longitudinale et transversale de l'élément de feuille (1).

6. Dispositif selon la revendication 4 ou 5, **caractérisé en ce que** les poches (5) présentent la forme d'un parallélogramme.

7. Dispositif selon la revendication 4 ou 5, **caractérisé en ce que** les poches (5) présentent la forme d'un trapèze.

8. Dispositif selon l'une quelconque des revendications 1 à 7, **caractérisé en ce que** les poches (5) sont constituées de bandes textiles et sont cousues le long de leurs bords (7, 8).

9. Dispositif selon l'une quelconque des revendications 1 8, **caractérisé en ce que** les poches (5) sont reliées les unes aux autres le long de leurs bords (8) s'étendant dans la direction longitudinale du corps d'insertion (3).

10. Dispositif selon l'une quelconque des revendications 1 à 9, **caractérisé en ce que** les compartiments sont formés par au moins une couture transversale (9) divisant les poches (5) qui s'étend transversalement aux bords transversaux (7) parallèles l'un à l'autre.

11. Dispositif selon l'une quelconque des revendications 1 à 9, **caractérisé en ce que** les compartiments sont formés par au moins une nervure transversale divisant les poches qui s'étend transversalement aux bords transversaux (7) parallèles l'un à l'autre.
